(19) Europäisches Patentamt
European Patent Office
Office européen des brevets

(11) Veröffentlichungsnummer: **0 074 475**
**B1**

# (12) EUROPÄISCHE PATENTSCHRIFT

(45) Veröffentlichungstag der Patentschrift: **16.01.85**

(51) Int. Cl.⁴: **C 08 F 2/50**, G 03 C 1/68, A 61 K 6/08

(21) Anmeldenummer: **82106103.3**

(22) Anmeldetag: **08.07.82**

(54) **Verfahren zur Photopolymerisation von Vinylverbindungen und photopolymerisierbares Material.**

(30) Priorität: **15.09.81 DE 3136484**

(43) Veröffentlichungstag der Anmeldung: **23.03.83 Patentblatt 83/12**

(45) Bekanntmachung des Hinweises auf die Patenterteilung: **16.01.85 Patentblatt 85/3**

(84) Benannte Vertragsstaaten: **AT CH DE FR GB LI SE**

(56) Entgegenhaltungen: **US - A - 4 071 424**

(73) Patentinhaber: **Kulzer & Co. GmbH, Philipp-Reis-Strasse 8, D-6393 Wehrheim (TS.)1 (DE)**

(72) Erfinder: **Schaefer, Roland, Dr., Merianweg 5, D-6382 Friedrichsdorf (DE)**

(74) Vertreter: **Heinen, Gerhard, Dr., Heraeusstrasse 12-14, D-6450 Hanau/Main (DE)**

EP 0 074 475 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Photopolymerisation von Vinylverbindungen in Gegenwart eines Photoinitiators aus

a) mindestens einem Photosensibilisator der allgemeinen Formel

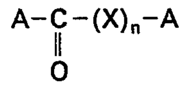

mit

$X=CO$, $C(R^1)(R^2)$ oder $C(R^3)(OR^4)$, worin $R^1$, $R^2$, $R^3$ und $R^4$ H oder Kohlenwasserstoffreste bedeuten,

n=0 oder 1,

A=gegebenenfalls substituierte Kohlenwasserstoffreste, die miteinander verbunden sein können und für n=1 und $X=C(R^1)(R^2)$ und für n=0 aromatische Reste sind,

und

b) mindestens einem Reduktionsmittel.

Die Photopolymerisation findet vielseitige, auch technische Anwendung, z.B. zur Härtung von Lacken und Überzügen, zur Herstellung von Druckplatten und beim Buchdruck.

Auch auf dem Dentalgebiet wird die Photopolymerisation angewandt. Photopolymerisierbare Materialien dienen zur Herstellung von Zahnfüllungen und -versiegelungen, von Kronen und Brücken und künstlichen Gebissen (s. z.B. die britische Patentschrift Nr. 569974 und die deutschen Offenlegungs- bzw. Auslegeschriften Nrn. 2315645, 2357324, 2910077 und 2914537).

In der britischen Patentschrift Nr. 1408265 werden photopolymerisierbare Materialien beschrieben, die als Photoinitiator ein Gemisch aus

a) mindestens einem Photosensibilisator der allgemeinen Formel

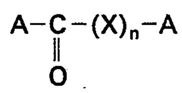

mit

$X=CO$, $C(R^1)(R^2)$ oder $C(R^3)(OR^4)$, worin $R^1$, $R^2$, $R^3$ und $R^4$ H oder Kohlenwasserstoffreste bedeuten,

n=0 oder 1,

A=gegebenenfalls substituierte Kohlenwasserstoffreste, die miteinander verbunden sein können und für n=1 und $X=C(R^1)(R^2)$ und für n=0 aromatische Reste sind,

und

b) mindestens einem Reduktionsmittel der allgemeinen Formel

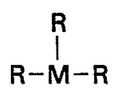

worin M ein Element der Gruppe VB bedeutet, das gegebenenfalls mit zwei Resten R einen Ring bilden kann,

enthalten und durch Bestrahlung mit sichtbarem Licht oder durch UV-Strahlen ausgehärtet werden können.

Als Beispiele für die Photosensibilisatoren werden u.a. Biacetyl, Benzil, p,p'-Dialkoxybenzil, Benzoin und Campherchinon, für die Reduktionsmittel Propylamin, Dimethylaminoäthylmethacrylat, N,N'-Dimethylanilin und Piperidin genannt.

Diese photopolymerisierbaren Materialien sind im Dunkeln lagerfähig und härten bei Bestrahlung rasch aus.

Aus der GB-PS Nr. 1465897 sind photopolymerisierbare Dentalmaterialien bekannt, die einen Photoinitiator aus

a) mindestens einem Photosensibilisator der allgemeinen Formel

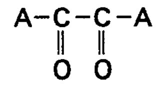

z.B. Benzil oder Campherchinon,

und

b) mindestens einem Reduktionsmittel, z.B. Dimethylaminoäthylmethacrylat,

enthalten.

Es ist die Aufgabe der Erfindung, einen Photoinitiator aus

a) mindestens einem Photosensibilisator und

b) mindestens einem Reduktionsmittel für die Photopolymerisation von Vinylverbindungen zu finden, der ein schnelles Aushärten bewirkt und zu einer guten Farbbeständigkeit der Polymerisate führt.

Die Aufgabe wird erfindungsgemäss dadurch gelöst, dass als Reduktionsmittel mindestens eine Verbindung der allgemeinen Formel

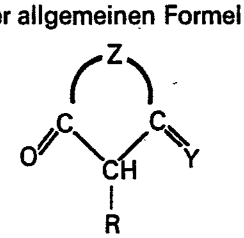

mit

Y=O, S oder $NR^1$,

Z=Alkylen mit 2 bis 3 C-Atomen, wobei 1 C-Atom durch eines der Heteroatome O und S oder 2 C-Atome durch die Heteroatome O, S und/oder N substituiert sein können,

R=Alkyl oder Aryl, gegebenenfalls substituiert,

$R^1$=H oder Alkyl,

verwendet wird.

Bevorzugte Reduktionsmittel sind die 5-Alkyl- und 5-Arylbarbitursäuren.

Als besonders geeignet haben sich die Photoinitiatoren aus Campherchinon und 5-Alkyl- bzw. 5-Arylbarbitursäuren erwiesen.

Die nach dem erfindungsgemässen Verfahren erhaltenen Vinylpolymeren und -copolymeren weisen keine Verfärbungen auf.

Das erfindungsgemässe Verfahren kann überall dort angewandt werden, wo monomere Vinylverbindungen bzw.solche Verbindungen enthaltende Materialien photopolymerisiert werden sollen.

Unter Vinylverbindungen werden alle gebräuchlichen äthylenisch ungesättigten Verbindungen verstanden, besonders Vinylchlorid und Acryl- und Methacrylsäureester ein- und mehrwertiger Alkohole, darunter auch die sogenannten

Urethanacrylate und -methacrylate und das aus der US-PS Nr. 3006112 bekannte Bis-GMA, das Reduktionsprodukt aus Bisphenol A und Glycidylmethacrylat.

Den Vinylverbindungen bzw. den diese Vinylverbindungen enthaltenden Materialien wird der Photo sensibilisator zweckmässigerweise in einer Menge von $10^{-2}$ bis 10 Gew.-%, bezogen auf die Vinylverbindungen, zugesetzt; bevorzugt wird eine Menge von $10^{-1}$ bis 5 Gew.-%. Das Reduktionsmittel kann in ebensolchen Mengen eingesetzt werden.

Besonders bewährt hat sich die Anwendung des erfindungsgemässen Verfahrens auf dem Dentalgebiet für die Herstellung sowohl von Zahnfüllungen und -versiegelungen als auch von Kronen, Brücken und künstlichen Gebissen.

In den folgenden Beispielen werden Vinylverbindungen enthaltende photopolymerisierbare Materialien und deren Polymerisation gemäss der Erfindung beschrieben.

Die Schichtdicke der erhaltenen polymeren Formkörper wird gemessen; sie dient zur Beurteilung bzw. zum Vergleich der Photoinitiatoraktivität.

*Beispiele 1 bis 3*

Eine Mischung aus

| | |
|---|---|
| 7 g | Bis-GMA, |
| 3 g | Triäthylenglykoldimethacrylat, |
| 30 g | feinteiligem Lithiumaluminiumsilicat (85 Gew.% der Teilchen mit Teilchengrösse <15 µm), und |
| X | Photoinitiator (s. Tabelle 1) |

wird in ein Glasröhrchen (Innendurchmesser 6 mm, Höhe 30 mm) gegeben, das mit einer Aluminiumfolie so abgedeckt wird, dass seitlich kein Licht einfallen kann. Die Mischung wird 2 min lang mit einer im Abstand von 17 cm angeordneten Wolframlampe (250 W/24 V) der F. Philips bestrahlt.

Der unpolymerisiert gebliebene Teil der Mischung wird entfernt und die Schichtdicke des erhaltenen polymeren Formkörpers gemessen.

In der Tabelle 1 werden die Art und Menge des Photoinitiators und die Schichtdicke angegeben.

*Tabelle 1*

| Beispiel | Initiator | Gew.-% | Schichtdicke (mm) |
|---|---|---|---|
| 1 | Campherchinon | 0,3 | 3,5 |
| 2 | Campherchinon<br>+<br>5-Butylbarbitursäure | 0,3<br><br>1,0 | >20 |
| 3 | Campherchinon<br>+<br>5-Butylbarbitursäure | 0,3<br><br>0,2 | 15 |

*Beispiel 4* (Vergleich)

Eine Beispiel 2 entsprechende Mischung wird in ein Glasröhrchen (Innendurchmesser 6 mm, Höhe 30 mm) gegeben, das vollständig mit einer Aluminiumfolie abgedeckt ist, so dass bei der Bestrahlung keine die Photopolymerisation auslösende Strahlung auf die Mischung einwirken kann. Dann wird das Röhrchen 2 min lang mit einer im Abstand von 17 cm angeordneten Wolframlampe (250 W/24 V) der F. Philips bestrahlt. Es tritt keine Polymerisation ein.

*Beispiel 5* (Vergleich)

Eine Beispiel 2 entsprechende Mischung wird in ein Glasröhrchen (Innendurchmesser 6 mm, Höhe 30 mm) gegeben und 5 min lang auf 120° C erwärmt. Es tritt keine Polymerisation ein.

*Beispiele 6 bis 12*

Eine Mischung aus

| | |
|---|---|
| 9,3 g | Bis-GMA, |
| 7,8 g | Triäthylenglykoldimethacrylat, |
| 6,9 g | Urethandimethacrylat, erhalten durch Reaktion von 1 mol Trimethylhexamethylendiisocyanat mit 2 mol 2-Hydroxyäthylmethacrylat, |
| 23,1 g | feinteiligem Siliciumdioxid, erhalten durch Flammhydrolyse, |
| 2,6 g | feinteiligem Aluminiumoxid, |
| 50,3 g | Splitterpolymerisat, erhalten durch Polymerisation einer Mischung aus Triäthylenglykoldimethacrylat und feinteiligem, durch Flammhydrolyse hergestelltem Siliciumdioxid, und |
| X | Photoinitiator (s. Tabelle 2), |

wird in ein Glasröhrchen (Innendurchmesser 6 mm, Höhe 30 mm) gegeben, das mit einer Aluminiumfolie so abgedeckt wird, dass seitlich kein Licht einfallen kann. Die Mischung wird 2 min lang mit einer im Abstand von 17 cm angeordneten Wolframlampe (250 W/24 V) der F. Philips bestrahlt. Der unpolymerisiert gebliebene Teil der Mischung wird entfernt und die Schichtdicke des erhaltenen polymeren Formkörpers gemessen.

In der Tabelle 2 werden die Art und Menge des Photoinitiators und die Schichtdicke angegeben.

*(Tabelle auf der nächsten Seite)*

*Beispiele 13 bis 16*

Eine Mischung aus

| | |
|---|---|
| 9,3 g | Bis-GMA, |
| 7,8 g | Triäthylenglykoldimethacrylat, |
| 6,9 g | Urethandimethacrylat, erhalten durch Reaktion von 1 mol Trimethylhexamethylendiisocyanat mit 2 mol 2-Hydroxyäthylmethacrylat, |
| 23,1 g | feinteiligem Siliciumdioxid, erhalten durch Flammhydrolyse, |
| 2,6 g | feinteiligem Aluminiumoxid, |
| 50,3 g | Splitterpolymerisat, erhalten durch Polymerisation einer Mischung aus Triäthylenglykoldimethacrylat und feinteiligem, durch Flammhydrolyse hergestelltem Siliciumdioxid, und |
| X | Photoinitiator (s. Tabelle 3), |

wird in ein Rohr (Innendurchmesser 6 mm, Höhe

*Tabelle 2*

| Beispiel | Initiator | Gew.-% | Schichtdicke (mm) |
|---|---|---|---|
| 6 (Vergleich) | Campherchinon | 0,2 | 3 |
| 7 (Vergleich) | Campherchinon + N,N'-Dimethyl-p-toluidin | 0,2<br>0,2 | 6,6 |
| 8 | Campherchinon + Methylmeldrumsäure (= Methylmalonsäureisopropylidenester) | 0,2<br>0,2 | 5,2 |
| 9 | Campherchinon + 1-Benzyl-5-phenylbarbitursäure | 0,2<br>1,0 | >20 |
| 10 | Campherchinon + 1-Benzyl-5-phenylbarbitursäure | 0,2<br>0,2 | 19 |
| 11 | Campherchinon + 5-Butylbarbitursäure | 0,2<br>1,0 | >20 |
| 12 | Campherchinon + 5-Butylbarbitursäure | 0,2<br>0,2 | 15 |

30 mm) aus Delrin®, einem Polyacetalkunststoff, gegeben und 20 s lang mit dem Wolfram-Halogen-Lichtgerät Translux der F. Kulzer bestrahlt. Der unpolymerisiert gebliebene Teil der Mischung wird entfernt und die Schichtdicke des erhaltenen polymeren Formkörpers gemessen.

Die Art und Menge des Photoinitiators und die Schichtdicke werden in der Tabelle 3 angegeben.

*Tabelle 3*

| Beispiel | Initiator | Gew.-% | Schichtdicke (mm) |
|---|---|---|---|
| 13 (Vergleich) | Campherchinon | 0,2 | 1,7 |
| 14 | Campherchinon + 5-Butylbarbitursäure | 0,2<br>1,0 | 3,0 |
| 15 (Vergleich) | p,p'-Dimethoxybenzil | 0,2 | 0,1 |
| 16 | p,p'-Dimethoxybenzil + 5-Butylbabitursäure | 0,2<br>1,0 | 1,0 |

Aus den in den Beispielen beschriebenen photopolymerisierbaren Materialien gemäss der Erfindung hergestellte Prüfkörper mit einem Durchmesser von 10 cm und einer Dicke von 2 mm werden gemäss der Norm für Zahnfüllungsmaterialien, ISO 4049, auf Farbbeständigkeit geprüft. Für die Bestrahlung wird das Gerät Suntest der F. W. C. Heraeus GmbH eingesetzt.

Nach einer Bestrahlungsdauer entsprechend der Norm zeigen die Prüfkörper keine sichtbaren Verfärbungen.

Bei unter Verwendung von Aminen als Reduktionsmittel hergestellten Prüfkörpern lässt sich unter den gleichen Bedingungen eine Verfärbung feststellen.

**Patentansprüche**

1. Verfahren zur Photopolymerisation von Vinylverbindungen in Gegenwart eines Photoinitiators aus

a) mindestens einem Photosensibilisator der allgemeinen Formel

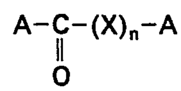

mit

X=CO, $C(R^1)(R^2)$ oder $C(R^3)(OR^4)$, worin $R^1$, $R^2$, $R^3$ und $R^4$ H oder Kohlenwasserstoffreste bedeuten,

n=0 oder 1,

A=gegebenenfalls substituierte Kohlenwasserstoffreste, die miteinander verbunden sein können und für n=1 und $X=C(R^1)(R^2)$ und für n=0 aromatische Reste sind,

und

b) mindestens einem Reduktionsmittel, dadurch gekennzeichnet, dass als Reduktionsmittel eine Verbindung der allgemeinen Formel

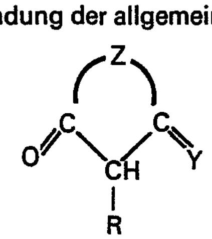

mit

Y=O, S oder $NR^1$,

Z=Alkylen mit 2 bis 3 Atomen, wobei 1 C-Atom durch eines der Heteroatome O und S oder 2 C-Atome durch die Heteroatome O, S und/oder N substituiert sein können,

R=Alkyl oder Aryl, gegebenenfalls substituiert,

$R^1$=H oder Alkyl,

verwendet wird.

2. Verfahren nach Anspruch 1, dadurch gekennzeichnet, dass das Reduktionsmittel eine 5-Alkyl- oder 5-Arylbarbitursäure ist.

3. Verfahren nach einem der Ansprüche 1 oder 2, dadurch gekennzeichnet, dass als Photosensibilisator eine Verbindung der allgemeinen Formel

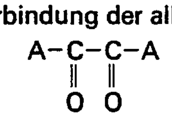

verwendet wird, worin die Gruppen A die in Anspruch 1 angegebene Bedeutung haben.

4. Verfahren nach einem der Ansprüche 1 bis 3, dadurch gekennzeichnet, dass der Photosensibilisator Campherchinon ist.

5. Verfahren nach Anspruch 4, dadurch gekennzeichnet, dass der Photoinitiator aus Campherchinon und 5-Alkyl- oder 5-Arylbarbitursäure besteht.

6. Vinylpolymere und Vinylcopolymere, erhalten durch das Verfahren nach einem der Ansprüche 1 bis 5.

7. Photopolymerisierbares Material, das mindestens eine Vinylverbindung und einen Photoinitiator aus

a) mindestens einem Photosensibilisator der allgemeinen Formel

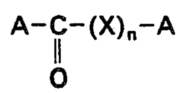

mit

$X=CO, C(R^1)(R^2)$ oder $C(R^3)(OR^4)$, worin $R^1$, $R^2$, $R^3$ und $R^4$ H oder Kohlenwasserstoffreste bedeuten,

n=0 oder 1,

A=gegebenenfalls substituierte Kohlenwasserstoffreste, die miteinander verbunden sein können und für n=1 und $X=C(R^1)(R^2)$ und für n=0 aromatische Reste sind,

und

b) mindestens einem Reduktionsmittel,

enthält, dadurch gekennzeichnet, dass als Reduktionsmittel eine Verbindung der allgemeinen Formel

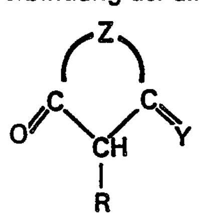

mit

Y=O, S oder $NR^1$,

Z=Alkylen mit 2 bis 3 Atomen, wobei 1 C-Atom durch eines der Heteroatome O und S oder 2 C-Atome durch die Heteroatome O, S und/oder N substituiert sein können,

R=Alkyl oder Aryl, gegebenenfalls substituiert,

$R^1$=H oder Alkyl,

verwendet wird.

## Claims

1. A process for photopolymerising vinyl compounds in the presence of a photoinitiator consisting of—

(*a*) at least one photosensitiser having the general formula

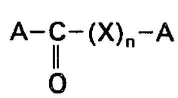

with $X=CO, C(R^1)(R^2)$ or $C(R^3)(OR^4)$, in which $R^1$, $R^2$, $R^3$ and $R^4$ denote H or hydrocarbon radicals, n=0 or 1, A=possibly substituted hydrocarbon radicals which may be connected to one another and are aromatic radicals for n=1 and $X=C(R^1)(R^2)$ and for n=0, and

(*b*) at least one reducing agent, characterised in that use is made, as a reducing agent, of a compound having the general formula

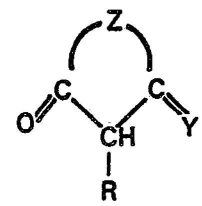

with Y=O, S or $NR^1$, Z=alkylene having 2 to 3 atoms, wherein 1 C atom may be substituted by one of the hetero atoms O or S or 2 C atoms by the hetero atoms O, S, and/or N, R=alkyl or aryl, possibly substituted, $R^1$=H or alkyl.

2. A process according to Claim 1, characterised in that the reducing agent is a 5-alkyl or 5-aryl barbituric acid.

3. A process according to Claim 1 or 2, characterised in that a compound having the general formula

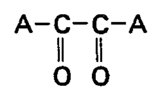

is utilised as a photosensitiser, the groups A having the significance specified in Claim 1.

4. A process according to one of the Claims 1 to 3, characterised in that the photosensitiser is camphor quinone.

5. A process according to Claim 4, characterised in that the photoinitiator comprises camphor quinone and 5-alkyl or 5-aryl barbituric acid.

6. Vinyl polymers and vinyl copolymers obtained by the process according to one of the Claims 1 to 5.

7. Photopolymerisable material which contains at least one vinyl compound and a photoinitiator consisting of

(*a*) at least one photosensitiser having the general formula

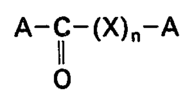

with $X=CO, C(R^1)(R^2)$ or $C(R^3)(OR^4)$, in which $R^1$, $R^2$, $R^3$ and $R^4$ denote H or hydrocarbon radicals, n=0 or 1, A=possibly substituted hydrocarbon radicals which may be connected to one another and are aromatic radicals for n=1 and $X=C(R^1)(R^2)$ and for n=0, and

(*b*) at least one reducing agent,

characterised in that use is made—as a reducing agent—of a compound having the general formula

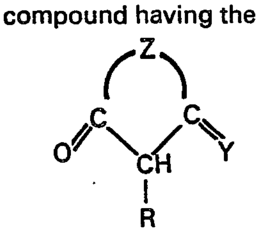

with Y=O, S or $NR^1$, Z=alkylene containing 2 to 3 atoms, wherein 1 C atom may be substituted by one of the hetero atoms O and S, or 2 C atoms by the hetero atoms O, S and/or N, R=alkyl or aryl, possibly substituted, $R^1$=H or alkyl.

## Revendications

1. Procédé pour la photopolymérisation de composés vinyliques en présence d'un photo-initiateur consistant en

a) au moins un photosensibilisateur de formule générale

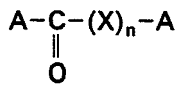

où

X=CO, $C(R^1)(R^2)$ ou $C(R^3)(OR^4)$, où $R^1$, $R^2$, $R^3$ et $R^4$ représentent H ou des restes d'hydrocarbures,

n=0 ou 1,

A=des restes d'hydrocarbures éventuellement substitués, qui peuvent être reliés ensemble et, pour n=1 et $X=C(R^1)(R^2)$ et pour n=0, des restes aromatiques,

et

b) au moins un agent réducteur,

caractérisé en ce que l'on utilise comme agent réducteur un composé de formule générale

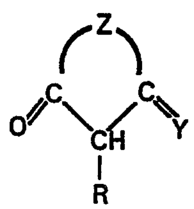

où

Y=O, S ou $NR^1$,

Z=alkylène avec 2 à 3 atomes, où 1 atome C peut être substitué par l'un des hétéroatomes O et S ou bien 2 atomes C peuvent être substitués par les hétéroatomes O, S et/ou N,

R=alkyle ou aryle, éventuellement substitué,

$R^1$=H ou alkyle.

2. Procédé selon la revendication 1, caractérisé en ce que l'agent réducteur est un acide 5-alkyl- ou 5-arylbarbiturique.

3. Procédé selon l'une des revendications 1 ou 2, caractérisé en ce que l'on utilise comme photosensibilisateur un composé de formule générale

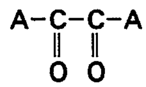

où les groupes A ont la signification indiquée dans la revendication 1.

4. Procédé selon l'une des revendications 1 à 3, caractérisé en ce que le photosensibilisateur est la camphoquinone.

5. Procédé selon la revendication 4, caractérisé en ce que le photo-initiateur consiste en camphoquinone et acide 5-alkyl- ou 5-arylbarbiturique.

6. Polymères vinyliques et copolymères vinyliques, obtenus par le procédé selon l'une des revendications 1 à 5.

7. Matériau photopolymérisable, qui contient au moins un composé vinylique et un photo-initiateur constitué par

a) au moins un photosensibilisateur de formule générale

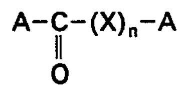

où

X=CO, $C(R^1)(R^2)$ ou $C(R^3)(OR^4)$, où $R^1$, $R^2$, $R^3$ et $R^4$ représentent H ou des restes d'hydrocarbures,

n=0 ou 1,

A=restes d'hydrocarbures éventuellement substitués, qui peuvent être reliés ensemble et, pour n=1 et $X=C(R^1)(R^2)$ et pour n=0, des restes aromatiques,

et

b) au moins un agent réducteur,

caractérisé en ce que l'on utilise comme agent réducteur un composé de formule générale

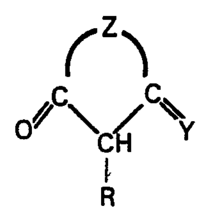

où

Y=O, S ou $NR^1$,

Z=alkylène avec 2 à 3 atomes, où 1 atome C peut être substitué par l'un des hétéroatomes O et S ou bien 2 atomes C peuvent être substitués par les hétéroatomes O, S et/ou N,

R=alkyle ou aryle, éventuellement substitué,

$R^1$=H ou alkyle.